# EUROPEAN PATENT APPLICATION

(11) **EP 1 122 316 A1**
(43) Date of publication of application: **08.08.2001**
(21) Application number: 98923194.9
(22) Date of filing: 29.05.1998
(51) Int. Cl.: C12N 15/82, C12N 5/04, A01H 5/00, A01H 5/10, A01H 4/00, C12P 21/06

(54) **PROCESS FOR OBTAINING TRANSGENIC PLANTS WHICH HAVE AN IMPROVED CAPACITY FOR THE UPTAKE OF NUTRIENTS AND TOLERANCE TO TOXIC COMPOUNDS WHICH ARE PRESENTS IN THE SOIL**

(71) Applicant: Centro de investigacion y Estudios Avanzados del Instituto Politecnico nacional, Irapuato, Ganajuato 36500 (MX)
(72) Inventor: HERRERA ESTRELLA, Luis Rafael, Irapuato, Ganajuato 36610 (MX)
(74) Representative: Flaccus, Rolf-Dieter, Dr.
(86) International application number: MX9800020
(87) International publication number: WO9963100

(57) **Abstract**

This invention refers to a method for obtaining plants by genetic engineering techniques, with improved capacity to synthesize, to accumulate and to exude organic acids. More specifically it refers to the production, of transgenic plants having an improved capacity to take up and excrete organic acids, providing them with a better uptake capacity of nutrients naturally present in the soil or added as fertilizers to the soil. These plants also have an increased capacity to tolerate the presence in the soil of certain toxic compounds such as aluminum. The transformation method implies the introduction of genes that increase the capacity of the plant to produce organic acids and involvess the following steps: a) preparation of a recombinant molecule comprising the coding sequence for an enzyme that produces organic acids, functionally bound to a promoter sequence active in plant cells and a transcription terminator functional in plant cells, b) transformation of plant cells with said recombinant molecule, c) the regeneration of transgenic plants from the transformed cells.

## Description

### BACKGROUND OF THE INVENTION

The invention relates to a method for obtaining transgenic plants having an increased capacity to synthesize and exude organic acids when compared with equivalent non-transformed plants, to a DNA molecule to produce such transgenic plants, the transgenic plants with increased organic acids synthesis capacity and excretion and use of the transgenic plants.

There are at least 17 mineral elements essential for plant growth and development. These elements include C, H, O, N, K, P, Mg, Ca, S, Fe, B, Mn, Cu, Zn, Mo, Cl and Ni, the micronutrients, for example molybdenum, are required at concentrations under one part per million, but if they are absent, the plant cannot complete its life cycle. Other nutriente such as nitrogen and phosphorus (macronutrients) are needed at high levels and reach concentrations of up to 3% of the total dry weight of the plant (Crawford, N.M (1994) In: Arabidopsis. Cold Spring Harbor Press pp. 1119-1146).

Nitrogen, phosphorus and Iron, are the nutrients most frequently limiting the yield of crops. These elements, besides being essential nutrients, act as environmental signals that can dramatically modify the physlology and development of plants, for example, by altering the growth rate and ramification of the roots in to relation to the extracellular concentration of these ions or by activating the expression of specific genes such as those encoding transporters and reductases (Goldstein, A.H (1991), Theor. Appl. Genet. 82:191-104).

Each nutrient has particular properties affecting its availability and uptake by the plant,, thus for example, although P and Fe may be present at suitable concentrations in the soil, they are often in the form of insoluble compounds, which are not readily available for uptake and use by the plants. The availability of P and Fe depends largely on the soil pH. Iron precipitates when combined with hydroxyl ions in alkaline soils, and phosphorus forms insoluble compounds by strongly binding to Ca and Mg in alkaline soils or to Mn, Fe and Al in acid soils.
To solve the problem of low nutrient availability in the soil,, farmers have recurred to the intensive application of fertilizers, on which about 10 billion dollars in production are spent each year in the United States (Glass, A. D.H (1989) "Plant nutrition: An introduction to current concepts. Jones and Bartlett, Boston, Massachusetts). Worldwide, over 140 million tons of nitrogen, phosphorus and potassium are provided to crops. However, only a small percentage thereof is taken up by plants and the rest is lost due to several reasons. In acid soils a great part of the P provided to the crops as fertilizer, reacts with Fe and Al ions, forming insoluble compounds not available to plants; this process is called phosphate fixation to the soil. In a recent study it has been suggested that in agricultural systems in which application of P to the soil is necessary to ensure plant productivity, the uptake of applied P by crop plants in a growing season is very low, because in the soil more than 80% of the applied P becomes immobile and unavailable for the plant due to adsorption, precipitation and conversion to organic forms (Holford I.C.R. (1997). Aust. J. Soil Res. 35:227-239).

A large part of the fertilizer used in the world, is lost due to soil washing during the rainy season, this fertilizer reaches the water tables and becomes a very serious pollution problem.
In relation to this last problem, the emphasis initially placed on plant nutrition to maximize production, has changed towards minimizing the loss of nutrients and to reducing harmful effluent from fertilizers into the environment (Mannetje, L. (1994). In Grassland and Society. Mannetje and Frame. Wageningen Press, Wageningen). For example, the Dutch Government passed laws in January 1998, to limit the use of Nitrogen and Phosphorus in agriculture. The use of these elements will have to be subjected to strict monitoring with respect to the nutritional status of the soil, to maintain crop production with minimum application of fertilizers. Not only in Holland, but in most countries, the development of cultivars with high nutritional efficiency is an urgent requirement in order to reduce the negative effect of many agricultural practices on the ecosystem (Loneragan, J.F (1997). Plant and Soil 196:163-174).
To cope with the inability of most plants to utilize insoluble forms of nutrients in the soil, several treatments have been developed. Among them, the following patented treatments can be mentioned: addition of organic acids (patent: US 5,593,947; January 14, 1997), the addition of chelated nutrients (patent: EFO 0 284 339; March 22, 1988) or inoculation with microorganisms that solobilize nutrients (see patent: US 5,026,4I7; January 25, 1991). In spite of being effective, these treatments are expensive and in many cases have to be repeated every year. Therefore, a better solution would be the availability transgenic plants with a higher efficiency in the uptake of nutrients, including those naturally existing in soil or those applied in the form of fertilizers.

Based on the possibility to obtain such plants , as shown in this invention, for the case of Phosphorus uptake, two large applications are foreseen: 1) The effective exploitation of natural Phosphorus reserves in soils affected by pH, in which this nutrient is unavailable for most plants; 2) to meet the phosphate requirements for crops with lower application of fertilizers.

Plant roots excrete a great variety of ions and organic substances, which affect the availability of nutrients in the soil. For Instance, the synthesis and excretion of chelating compounds, such as organic acids and phytochelatins, have been proposed as a mechanism for the mobilization of different elements, such as P, Fe, Mn and Zn from insoluble compounds present in the soil (Marschner, H. (1995) Mineral Nutrition of Higher Plants. Academic Press, San Diego, CA). In the context of the current invention, exudation and excretion-are considered the same process that we define as the process by which plants, through their roots send organic and inorganic compounds into the soil.

The chemical nature of the substances excreted by roots depends on several factors: inter- and intra-species genetic differences, age of the plant and nutritional status. The components identified in root exudates can be grouped in two large classes: mucilaginous substances and organic solutes. The organic solutes include sugars, organic acids, amino acids and phenolic compounds.

Organic acids are very versatile molecules that participate in different physiological processes in all living organisms including plants. The biosynthesis of these compounds is a ubiquitous process in living organisms. In animals their participation as precursors of important metabolic pathways such as the Krebs cycle or the glyoxylate cycle, their transport mechanisms and the enzymes they interact with, are well known. In contrast, little information is available for plants in this respect (Srere, P.A. (1992). Curr. Top. Cell. Reg. 33:261-275).

In the context of the present invention, organic acids is used as a general term that includes organic acids and their corresponding bases (e.g. citric acid and citrate), since depending upon the pH these molecules may interconvert.

The excretion of organic acids has been correlated with the capacity of some plant species, such as rape (*Brassica napus*) and white lupin (*Lupinus albus*), to solubilize phosphate from insoluble compounds such as aluminum or iron phosphates or even phosphoric rock. In both cases an increment in organic acid excretion is observed, as a response to stress caused by phosphate deficiency (Hoffland et al. (1989) plant and Soil 113:161-165).

Based on these correlations, it has been proposed that the natural exudation of organic acids by *Brassica napus,* is an effective strategy to increase phosphate uptake from phosphoric rock. In the case of *Lupinus albus,* under conditions of low phosphate availability, the formation of specialized roots, termed proteoid roots, is induced. These roots exude large quantities of citric acid, allowing this plant species to provoke the solubilization of phosphate from insoluble compounds. In addition to this correlation, organic acids, such as citrate, malate, oxalate, tartrate, malonate and lactate, have been used since 1950 by soil chemists to extract phosphate from insoluble compounds present in the soil. Of all the organic acids mentioned above, citrate is the most effective to dissolve insoluble phosphate compounds.

Another important problem affecting nutrient uptake from the soil is the presence of toxic compounds, such as certain soluble forms of aluminum or other metals that can interfere with the growth and function of plant roots.

Aluminum (Al) is the most abundant metal in the earth's crust (comprising about 7% of its mass) and is found in soils primarily in the form of insoluble aluminum-silicates and oxides. However, when solubilized in acid soils, Al (primarily in the form of Al³⁺) is highly toxic to many crops. Al toxicity is considered to be the major limiting factor for plant productivity on acid soils. Soil acidification can develop naturally when basic cations are leached from soils, but it can also be considerably accelerated by certain farming practices and by acid rain. Acid soils account for about 40% of the arable land worldwide and are particularly abundant in tropical and subtropical regions of the world (e.g. 850 million ha. in tropical America) (Foy, CD. et al. (1978). Annu. Rev. Plant Physiol. 29: 511-66).

A common practice in order to maintain the agricultural productivity in acid soils, is the application of calcium hydroxide (lime) or calcium sulfate (gypsum, CaSO₄.2H₂O) to increase the soil pH (e.g. see US 5,628,811; May 13, 1997). Although these types of soil treatments have been successful, they do not represent a viable solution for many farmers since they do not have the economic resources to obtain and apply them, and in addition their application may lead to undesirable effects such as river pollution.

In plants, aluminum produces general toxic symptoms that are similar to nutrient deficiencies (Bennet, RJ et al. (1986). J. Plant Soil. 3: 11-17.). Decreased mineral nutrition appears to be due mainly to the inhibition of root development caused by the targeted action of Al at the plant root tip (Ryan, PR et al. (1993). J. Exp. Bot. 44: 437-446), In simple nutrient solutions, micromolar concentrations of Al can begin to inhibit root growth within 60 minutes.
Several studies have shown the existence of considerable *inter-* and *intra*-species genetic variability regarding the tolerance of plants to aluminum toxicity (Baligar, VC et al. (1993), Plant and Soil. 150: 271-277.). Although several hypotheses have been proposed to explain the genotypic differences that render plants tolerant to aluminum, strong evidence suggests that in several plant species, tolerance occurs by aluminum exclusion from the root tip (Delhaize E. et al (1993). Plant Physiology. 103: 685-593). In fact, it has been observed that sensitive wheat cultivars accumulate three-to eight-fold more aluminum than tolerant cultivars in their root apex (Tice, KR. Et al. (1992). Plant Physiol. 100: 309-318).

Aluminum-tolerance in wheat (*Triticum sp*), Corn (*Zea mayz*) and sweet pea (*Vicia faba L*.) has been correlated with an increased capacity to release organic acids, such as malic and *citric* acids (Miyasaka SC et al. (1991). Plant Physiology. 91: 737 743; Delhaize AND et al. (1993). Plant Physiology. 103: 695-702). Excreted organic acids have been proposed to confer tolerance by complexing with Al³⁺ outside the plasma membrane, preventing its uptake (Miyasaka SC et al. (1991). Plant Physiology. 91:737-74312).

In agreement with the proposed role of organic acids as chelating agents that prevent aluminum toxicity, it has been shown that addition of organic acids, such as citric and malic acid, to the nutrient solution where plants are grown, significantly decreases the toxic effect of aluminum. These experiments have also shown that citric acid is more effective than succinate or malate in reversing Al toxicity. (Hue, NV et al. (1986). Soil. Sci. Soc. Act J. 50: 28-34; Barlett, RJ. and Riego, DC. (1972). Plant Soil. 37: 419-423.).

The affinity of citrate for biologically important cations has been known for several years. In pioneer studies directed by D.H. Sieling of the University, of Massachusetts, the capacity of several substances forming part of the soil's organic component to avoid the precipitation of phosphate caused by iron and aluminum, was compared. Among the results. obtained in this work; it was found that citrate strongly binds to iron and aluminum, inhibiting phosphate precipitation at different pH values, this interaction being more efficient compared to the effect promoted by other organic acids. In the mentioned reaction, one millimole of citrate interacts with one milimole of Aluminum reducing the precipitation of Phosphate up to 100% in a range of 4.0 - 9.0 pH units.

It has been suggested that the effectiveness of citrate to interact with cations, is mainly determined by the negative charges present in their structure, which promote the formation of highly stable organometallic complexes.

Recent evidence suggests that organic acids, especially citrate, play a fundamental role in aluminum-tolerance in plants (Miyasaka at al. (1991). Plant Physiology 96:737 743. De la Fuente et al. (1997) Science 276:1566-1568) and heavy metals (Yang et al. (1997) Plant and Soil 196:271-176), as well as in the uptake of nutrients, such as Phosphorus, Iron and Nickel (Jones, D. L. and P.R Darrah (1994) Plant and Soil 166:247-257).

The enzymes that synthesize organic acids and their corresponding genes, have been extensively studied in bacteria and animals. Among the most studied are citrate synthase and malate dehydrogenase, which have been characterized in great detail and their corresponding genes from an important number of bacteria, animals and some plants have been isolated and sequenced. For the case of Citrate Synthase more than 300 genes have been cloned and characterized, while for the Malate Deshidrogenase more than 500 genes have been cloned and sequenced.

Citrate synthase, catalyzes the synthesis of citrate by condensation of the methyl group of acetyl coenzyme A and the carbonyl group of oxalacetate. The amino acids that form the active site of this enzyme are known and are conserved in all analyzed enzymes including those of Rickettsia and Arabidopsis (Alter et al: (1990). Biochemistry. 29:7557-7563). The citrate synthases of the gram negative bacteria Escherichia coli, Acinctobacter anitratum and Pseudomonas aeruginosa, are allosteric enzymes whose activity is strongly inhibited by NADH and share a homology of about 75% in their amino acid sequence (Alter et al. (1990). Biochemistry. 29:7557-7563).

In plants, two isoforms of Citrate Synthase have been identified, one located in the mitochondria and the other present in the glyoxysome. The analysis of the complementary DNAs that code for the mitochondrial enzymes of Arabidopsis thaliana and potato (Solanum tuberosum), showed homology with the pig and yeast enzymes, whereas the glyoxysomal citrate synthase of Cucumber seems to be more related to bacterial counterparts. Both isoforms are immunologically different and apparently not subjected to allosteric regulation (Kato et al. (1995). Plant Mol. Bio. 27:377-390).

The feasibility of producing transgenic plants that express alien genes has been thoroughly, demonstrated. Successful expression of foreign genes in transgenic plants has been achieved for genes of plant origin, as well as bacterial, viral and animal origin.

There are different mathods of genetic transformation for obtaining transgenic cells from which fertile transgenic plants can be regenerated. Among these methods are, the electroporation of protoplasts or intact tissues, the co-cultivation of cells or intact tissues with non-oncogenic Agrobacterium tumefaciens strains and bombardment with microparticles or biolistics are them most commonly used.

This patent relates to the demonstration that plants can be genetically engineered to provide them with a higher capacity to synthesize, accumulate and exude organic acids. The invention then provides a method to obtain transgenic plants with an increased capacity to synthesize and exude organic acids comprising the following steps:
a) The preparation of a genetic construct comprising one or more enzymes that synthesize organic acids, functionally linked to a promoter sequence transcriptionally active in plants and a transcription termination sequence active in plant cells;
b) The transformation of plant cells with the previous genetic construction; and
c) The regeneration of transgenic plants starting from the transformed cells.

### Description of the Invention

Acknowledging the importance that nutrient availability has for agricultural production, the fact that many of the nutrients present in soil or added as fertilizers are converted into insoluble forms and that certain ions present in the soil can be toxic to plant roots, it is highly desirable to Obtain plants with an improved nutrient uptake capacity from insoluble compounds present in the soil. Therefore, the application, of recombinant DNA and Genetic Engineering techniques for the production of such plants is essential.

Since there is evidence strongly suggesting that high levels of organic acid exudation facilitate nutrient solubilization and absorption (particularly in the cases of phosphorus and iron), the production of transgenic plants with an increased capacity to produce and excrete organic acids is highly desirable.

In addition, if has been proposed that exudation of organic acids is a mechanism used by some plants to combat the toxic effects of some elements present in the soil, as in the case of the aluminum in acid soils. Therefore, transgenic plants that have a high capacity for the synthesis and excretion of organic acids, would not only have a better capacity to use nutrients biologically not available in the soil, but also to tolerate toxic concentrations of some heavy metals such as aluminum.

In the present invention, a method for obtaining transgenic plants with an increased capacity for production, accumulation and exudation of organic acids is described. In particular, the invention refers to the overproduction of citric acid, however, the present invention is not limited to the overproduction of citric acid, since the overproduction of other organic acids, such as malic acid and oxalic acid among others is also possible.

One of the aspects of this invention, describes the construction of a recombinant DNA molecule encoding the enzyme citrate synthase, linked to a promoter sequence and a transcription termination sequence both functional in plants. The present invention refers, but is not limited, to the use of the gene encoding Citrate Synthase, since it is possible that the use of other genes encoding enzymes that synthesize other organic acids such as those capable of sinthesizing malic and oxalic acids would also be effective.

In plant cells, the synthesis of citric acid takes place primarily in the mitochondria, where the first reaction of the Krebs cycle is carried out by the enzyme citrate synthase.
Since citric acid synthesis is part of a complex cycle, where the flow of carbon structures not necessarily accumulates in just one of its components end is subjected to complex regulatory mechanisms, it is highly desirable to carry out the invention compartamentalizing a citrate synthase in a subcellular compartment different to the mitochondria. This strategy would avoid the additional citric acid being converted into other components of the Krebs cycle. Therefore, an important aspect of this invention is the description of a method for obtaining transgenic plants where the Citrate Synthase is located in a subcellular compartment different to the mitochondria, such as the cytoplasm or the chloroplast. Thus, the recombinant DNA molecule described in this invention. can contain a signal or transit peptide directing the citrate synthase to a specific compartment of the cell.

The present invention has advantages over the existing technology since transgenic plants obtained by this method have a better capacity for nutrient uptake and to tolerate toxic compounds without the necessity of using chemical treatments of the soil or the use of nutrients associated with chelating compounds. Industry or farmers can use the transgenic seeds to establish crops, reducing their production costs, in terms of treatmente of the soil or addition of fertilizers, or increasing the productivity thereof in acid soils containing toxic levels of aluminum or in those not having available nutrients.

Part of this invention, that describing the method for obtaining transgenic plants that overproduce citrate and the increased capacity of these plants to tolerate toxic concentrations of aluminum, has been previously published (De· la Fuente et al, Science 277, 1566-1568).

### Detailed Description of the Invention.

The present invention is directed at the production: of transgenic plants with an increased capacity of synthesis and exudation of organic acids.

According to one of the aspects described in this invention, recombinant molecules and methods are described which allow the production of transgenic plants with an increased capacity of synthesis and exudation of organic acids.

The preferred form of organic acid in this invention is citric acid, since it has been demonstrated as one of the most effective to solubilize and facilitate absorption of nutrients of the soil, and to eliminate aluminum toxicity.

In this invention we refer to Citrate Synthase as any enzyme able to synthesize citric acid. The present invention refers, but it is not limited to Citrate Synthase, since it is possible to use other genes encoding enzymes able to synthesize other organic acids, to the same effect. Citrate Synthase or any other enzyme that synthesizes organic acids, should have kinetic parameters compatible with the biochemical and physiological systems of the plant of interest.

The gene or the coding part of a gene for an enzyme that synthesizes organic acids can be derived from a complementary DNA molecule, from genomic DNA or can be synthesized chemically, totally or partially. The desired gene can be obtained from any microorganism, any plant or any animal.

In general, the gene or part of the same will bo derived from native sequences of some organism. In the case of enzymes able to synthesize organic acids, and in particular Citrate Synthase, a great number of genes have previously been identified and characterized, including the determination of their nucleotide sequence.

To achieve the necessary levels of expression of the cloned gene in the appropriate tissue, it is desirable, for genes of plant origin to be placed in an expression cassette including a promoter sequence functional in plants,, the coding sequence of the gene of interest and a transcription termination sequence functional in plants. Additionally the cassette may include a sequence coding for a transit peptide that directs the enzyme to a specific compartment of the cell. The transit peptide and the corresponding processing signals can be derived from any plant protein synthesized in the cytoplasm and translocated to the subcellular compartment of interest, either the plastid or the mitochondria. For example to locate a citrate synthase into the plastid, transit peptide sequences from genes encoding the small subunit of the ribulose bisphosphate carboxylase or chlorophyll a/b proteins, can be used (Van den Broek et al, Nature. (London) 313, 358-363 (1985)).

To target bacterial enzymes into the cytoplasm, it is not necessary to add any transit peptide, .since proteins that do not contain specific targeting sequences, will remain in the cytoplasm in a natural way.

In the case of enzymes coming from eukaryotes, if they are located in a different subcellular compartment to the cytoplasm, it is necessary to remove their natural transit peptide to locate them in the cytoplasm.

For the case of genes not of plant origin, in addition to a functional promoter, the use of an initiation codon context optimal for translation in plants or translation enhancers might be desirable in some cases to achieve high levels of expression. The promoters that can be used include promoters of the plant to be transformed, promoters of other plants or of any origin that are functional in the target plant and direct a constitutive, inducible or tissue specific expression. For example, promoters derived from the Ti plasmid of Agrobacteriun tumefaciens such as the promoters of the Octopine Synthetase, Nopaline Synthetase or Agropine Synthetase can be used. Viral promoters, such as the 35S promotor of the cauliflower mosaic virus or those derived from geminiviruses can also be used.

The temporal, inducible or tissue specific tissue expression can be achieved by using promoters or regulatory sequences that have the desired expression specificity. Although we have found that the invention works using constitutive promoters, it could be desirable to consider the use of root-specific promoters or those that are activated by stress caused by the phosphate or iron starvation. For example, the promoter of some genes encoding phosphate transporters, have been shown to be specifically expressed in the epithelium of the root and to be induced when little phosphate is available (Muchhal U.S., et al., Proc. Natl. Acad. Sci. 93: 10519-10523).

The transcription termination sequence can be derived from the same gene from which transcription promoter sequence was obtained or from a different gene. The transcription termination or 3' sequence can be derived from genes contained in the T-DNA of the Ti plasmid of Agrobacterium, the cauliflower mosaic virus or plant genes. For example, the nopaline synthase transcriptional termination sequence is frequently used in genetic engineering of plants.

The different nucleic acid sequences that form part of the recombinant DNA molecule can be joined by conventional methods described in the literature. The sequences should be cloned and joined in the orientation and correct order to achieve the functional expression in plant cells.

In accordance with one of the aspects of this invention, the plants of interest are transformed with a recombinant DNA molecule that encodes at least one enzyme able to synthesize organic acids. In the development of the recombinant molecule, the different nucleotidic sequences that form part of it, could have been subject to different processing, such as ligation, digestion with restriction enzymes, in vitro mutagenesis, oligonucleotide addition or modification by means of the polymerase chain reaction (PCR). Therefore, those components of the recombinant molecule before being joined could be subject to deletions, insertions or internal modifications.

As the recombinant molecule is derived from components that originate from different organisms and that have been isolated, purified or synthesized, it is not a molecule that exists as such in nature.

Depending on the transformation method used to introduce the recombinant molecule to the plant cell of interest, the inclusion of other sequences of DNA may be necessary. For example, it is necessary to include a cloning vector that allows replication in E. coli and. When Agrobacterium is going to be used as a transformation method an origin of replication functional in this bacteria and T-DNA borders are required. A gene that serves the function of a dominant selectable marker (such as those confering resistance to antibiotics or herbicides) is also generally included to allow the selection and identification of the colls that incorporated, in a stable way, the recombinant molecule in their genome.

The recombinant molecule can be introduced into the desired plant cell by any method of genetic transformation of plants. These methods include, but are not limited to, the following transformation methods: the transformation system mediated by the Ti plasmid of Agrobacterium tumefaciens, electroporation, microinjection and biobalistic or microparticle bombardment,

To use the genetic transformation system mediated by the Ti plasmid of Agrobacterium to introduce the recombinant molecule into the genome of plant cells, it is necessary to include the border sequences of the T-DNA, in such a way that they are present at both ends of the gene coding the enzyme that synthesizes organic acids and the dominant selectable marker gene. The use of disarmed Agrobacterium strains, those in which the genes responsible for tumor formation have been removed, but maintain the capacity to transfer DNA to plant cells, allows the regeneration of transgenic plants containing the recombinant molecule of interest.

The recombinant DNA molecule can be introduced into the genome of any plant species, including both monocots as well as dicots. Of special interest, is the introduction of the recombinant molecule into vegetable species that by nature have a low capacity to solubilize phosphorus and iron or are susceptible to aluminum toxicity. Representative examples of such vegetable species include but are not limited to corn (Zea mays), rice (Oryza sativa), wheat (Triticum sp), sorghum (bicolor Sorghum), soya (Glycine max L.), tobacco (Nicotiana tabacum), tomato (Lycopersicum sculetum), papaya (Carica papaya) and potato (Solanum tuberosum).

After the transformation of the tissue, plant cells or protoplasts derived from the plant of interest, transgenic plants that contain in their genome the recombinant molecule are regenerated. These transgenic plants are able to transfer in a stable way the recombinant molecule to their offspring, be this derived from seeds, cuttings, tubers or any other reproductive structure.

When growing these plants in the field, higher productivity can be obtained in soils that have restricted quantities of phosphorus. Better production will also be obtained in acid soils having toxic concentrations of aluminum. In cases where, phosphorus is applied as fertilizer, savings will be obtained in the quantity of fertilizer needed to obtain good productivity.

### Experimental data (Examples).

The present invention will be illustrated in the examples that are described next, which in no way are intended to limit the present invention.

### Example 1

Expression of the Citrate Synthase of Pseudomonas aeruginosa in the cytoplasm of transgenic plants of Tobacco.

### 1. Selection of genes to be used

To obtain plants that overproduce organic acids, genes that code for enzymes having the capacity to synthesize organic acids, were selected. One of these genes, the gene of Pseudomonas aeruginosa coding for the enzyme Citrate Synthase, was selected (Donald et al (1989), J. Bacteriol. 171: 5542-5550). This enzyme synthesizes citric acid using as substrates oxaloacetate and acetyl Coenzyme A.

To avoid that the citric acid, synthesized by the bacterial Citrate Synthase, is transformed into other components of the Krebs cycle, the cytoplasm was selected as the subcellular compartment in which to express this enzyme.

### 2. Construction of the recombinant molecule 35SCSb for the expression of Citrate Synthase of Pseudomonas aeruginosa in plants.

### I. Construction of the expression vector pB2

To achieve the expression of the. Pseudomonas aeruginosa Citrate Synthase coding sequence, the expression vector pB2 was constructed. To that end, the cab 80 promoter fragment of pGV1511 (Jofre Garfias et al (1997), plant Cell Reports 16: 847 852), defined by the restriction sites Hind III and Bam HI, was substituted by the Hind III Bam HI fragment of pBI 525 (Datla et al. (1993), Plant Science 94; 139 149.), which contains the double 35S CaMV promoter, the translation enhancer. region of the alfalfa mosaic virus and a translation initiation codon (as part of the Nco I site). The substitution of the promoter region of the pGV1511 vector for that of pBI 525 was verified by means of double restriction analysis using the Xba I and Hind III sites.

### II. Construction of the recombinant molecule p35SCSb

The Pseudomonas aeruginosa Citrate Synthase coding sequence starting at amino-acid number 9, corresponding to the gltA sequence limited by the Bel I and Bam HI restriction sites, was mobilized from the pPKB vector (Donald et al, (1989), J. Bacteriol. 171: 5542 5550) into the pB2 Bam HI site. The orientation of the cloned fragment was determined by analysis of the fragment released by a double restriction using the Xba I and Bam HI enzymes. Cloning the Bcl I Bam HI fragment from gltA in the correct orientation in pB2, results in an in frame translational fusion with the initiation codon present in the expression cassette of pB2. As a consequence of this fusion, the first 8 aminoacids of the bacterial citrate synthase are modified changing from the native sequence Met-Ala-Asp-Lys-Lys-Ala-Glu-Leu, into Met-Ala-Ser-Arg-Pro in p35SCSb; the rest of the citrate synthase sequence remains the same.

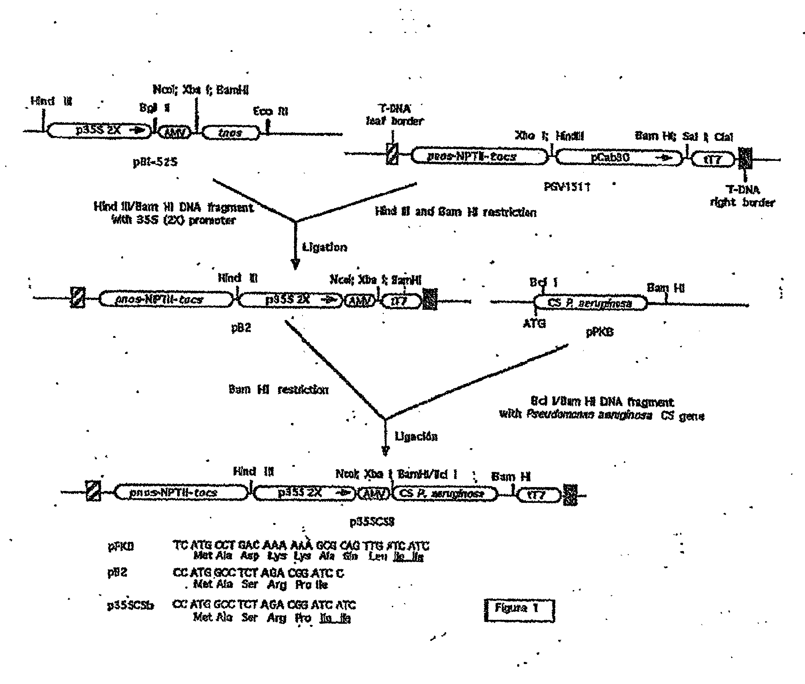

To verify, the reading, frame and the absence of modifications in the sequence of the recombinant molecule p35SCSb, the sequence of 600 base pairs was determined by the method of Sanger, starting from the Xba I site in p35SCSb. Figure 1 illustrates the steps followed for obtaining of p35SCSb starting from its different components.

### 3. Obtaining transgenic plants that contain in their genome the recombinant molecule 35SCSb.

The p35SCSb plasmid was conjugated from E. coli to the Agrobacterium strain LB4404 (Hoekema A. et al. (1983). Nature 303, 179-180) using the triparental conjugation system that uses the helper plasmid pRK2013 (Lam S.T. et al. (1985), Plasmid 13, 200-204). The recombinant molecule was introduced into the genome of tobacco cells (Nicotine tabacum L. var. xanthi) using the leaf disk transformation method (Horsh R. B. et al (1985), Science 227, 1229 1232, from which transgenic plants were regenerated. The regenerated plants, denominated CSb plants, were selected by growth in selective medium containing 50 micrograms of kanamycin per millilitre of cultivation medium.

### 4. Analysis of the CSb plants

To confirm that the CSb plants contained the recombinant DNA molecule in their genome, genomic DNA was extracted from leaves of kanamycin-resistant plants by conventional methods, digested with Hind III and Bam HI, subjected to 1% agarose gel electrophoresis, transferred to nylon membranes and hybridized with a probe specific, for the Pseudomonas aeruginosa citrate synthase gene. Over 95% of the kanamycine resistant plants contained the expression cassette of the Citrate Synthase of P. aeruginosa.

To verify that the expreesion cassette was functionally transcribed in the CSb plants, total RNA of these plants was extracted using conventional techniques and subjected to Northern-type RNA hybridization analysis using a fragment of the P. aeruginosa citrate synthase coding sequence as a probe. It was found that around 80-85% of the plants contained detectable levels of RNA messenger corresponding to the bacterial Citrate synthase. The levels of RNA messengers detected for each line were variable as is usually expected in plant transformation experiments. This variation in the expression levels of alien genes in plants depends mainly on the position of insertion of the foreign DNA in the genome of the target plant and it is known as the position effect.

Homozygote plants containing a single copy of the recombinant molecule were selected for further analysis. Under greenhouse conditions, no obvious phenotypical differences were observed between plants containing the p35SCSb T-DNA and non-transformed control plants, besides the observation that some transgenic plants produced more biomass and a bigger quantity of seeds than the controls.

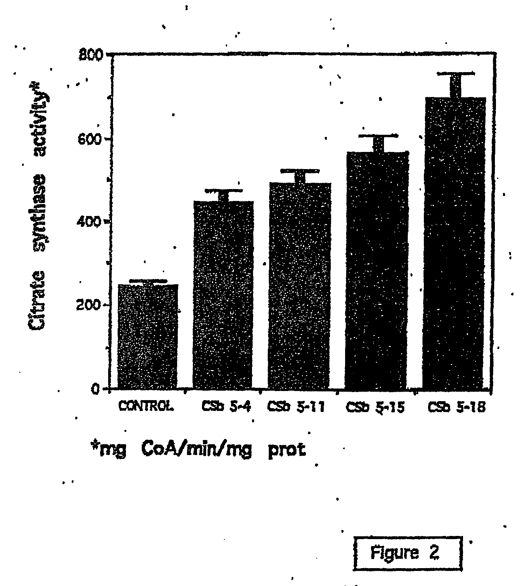

Biochemical analysis of CSb transgenic lines (Srere, P. (1969). Enzymol, 13:3 22) showed that several of them contained high levels of Citrate Synthase activity as compared to that present in control plants. Your lines were selected with activity levels of Citrate Synthase, between two and three times bigger than the control (see figure 2). The presence of the bacterial CS in CSb lines was confirmed by Western type analysis using an antiserum (Donald, J.L, et al. (1989.). Journal of Bacteriology. 5542-5550) which does not recognize the plant enzyme. Densitometric analysis of the western blot showed a good correlation between the level of bacterial CS detected and the increment in Citrate synthase activity in the different CSb lines. This demonstrates that the increased Citrate Synthase levels in CSb plants is due to the expression of the recombinant molecule encoding the P. aeruginosa Citrate Synthase.

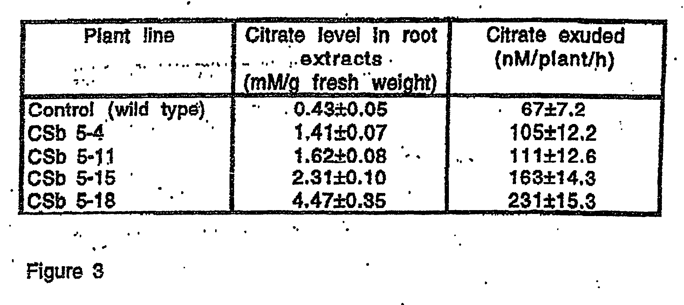

To determine whether the expresion of citrate synthase in the cytoplasm of plant cells causes an increment in citrate content, total root extracts of CSb lines were analyzed by high pressure liquid chromatography and compared with those of control plants. To determine the content of citrate in root extracts, 1, gram of tissue was ground in liquid nitrogen and extracted with 10 milliliters of boiling ethanol. The extract was centrifuged to 1000 rpm for 10 minutes and the supernatant filtered in a millipore filter with a 45 micron pore size. The content of organic acids was determined according to method of Picha. (1985, J. Agric. Food Chem. 33: 743-745). It was found that the lines expressing the p35SCSb construction, showed citrate levels in their roots up to 10 times higher that those of control plants (see figure 3).

It has been suggested that exudation, rather than intracellular accumulation of citrate, is the direct cause of the capacity of certain plants to dissolve and use insoluble form of certain nutrients or to tolerate toxic concentrations Aluminum. Thus, we examined whether an increment in citrate synthesis in CSb plants, could lead to an increment in its excretion. To quantify citrate excretion levels in the roots of CSb and control plants, 50 seedlings of each line were germinated in semisolid media and then transferred to sterile water for 12 hours; the quantity of citrate exuded into the water was determined by the high pressure chromatography method of Picha. It was found that the selected CSb lines had levels of citrate exudation up to 4 times higher than control plants (see figure 3). The chemical identity of the compound that is exuded in an increased way in CSb lines was confirmed to be citrate by mass spectrometry.

### Example 2

Properties acquired by transgenic plants containing in their genome the recombinant molecule that codes for the Citrate Synthase of Pseudomonas aeruginosa.

### I. Higher efficienoy in dissolution and uptake of phosphate.

With the purpose of simulating the nutritional conditions prevailing in a Vertisol soil (a soil with alkaline pH in which a high percentage of the phosphate forms insoluble compounds), we decided to use as a substrate a soil comprising sand:lime in a 1:1 proportion with a pH of 8.2. Chemical analysis of this mixture showed that it contained less than 5 parts per million (ppm) of Using phosphate sodium phosphate (NaH2PO4), two phosphate regimes were incorporated into this soil mixture, one at a concentration of 22 ppm and the other at 44 ppm. Sodium phosphate was incorporated into the soil as a single application from the outset of the experiment. Sodium phosphate is an easily available phosphorus source for plants, but of which a high percentage becomes insoluble under the conditions employed.

The remaining nutrients necessary for the growth of the plants were supplied daily in aqueous solution during the course of the experiment which lasted 6 months.

The plant material used consisted of two transgenic lines termed CSb 5-4 and CSb 5-18 that overexpressed the gene of the citrate synthase of P. aeuroginosa and wild type (1522) used as control.

The plants were placed in polyethylene bags containing 2 kilograms of soil with the corresponding phosphorus treatment. A random distribution of the plants was designed in the greenhouse, forming blocks for each treatment and consisting of 24 repetitions.

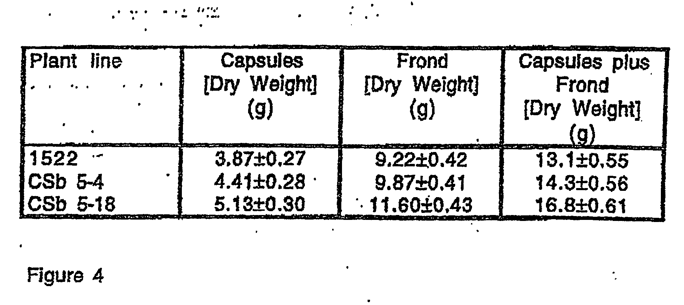

With the intention of monitoring the development of the plants in a more stringent manner, sampling was carried out in 3 development stages of the plants: vegetative growth, bloom and seed formation. At each stage 8 plants were analyzed to measure the following agronomic variables: height of the plant, leaf area, fresh weight of the plant, dry weight of the plant, dry weight of the root. The number of flowers (for the bloom stage) and total number and dry weight of capsules or fruits (for the seed formation stage) were also determined. Analysis of the data obtained for each of the treatments and each one of the analysed variables, revealed that a correlation exists among them, that is to say that taller plants have higher leaf area and higher dry weight. Due to this correlation and the observation that the dry weight represents this in a more direct way, the amount of biomass accumulated by each line at the end of the experiment is presented (figure 4), because it represents the total biomass aocumulated by the plant during its entire life cycle. The dry weight of the capsules, stem and total was determined in the following way:
a) Total dry weight of capsule (includes the biomass accumulated in the seeds).
   The collection of the capsules was carried out in fully senescencent stage of the plant, once the grain filling had concluded. Harvesting was carefully carried out dividing the capsules from the peduncle base and depositing them in paper bags. The bags were suitably labeled and placed in a Heraeus Baureihe 6000 stove at a temperature of 70°C for 72 hours. Dry material was weighed in an Mettler PE 360 analytic balance and the data obtained subjected to variance analysis (ANOVA) and means comparison with the method of Tukey.
b) Dry weight of the frond (includes the total biomass of stalks and leaves).
   The leaves were harvested from the petiole base and were completely included in rag paper bags. The stalk was divided from the crown (the stalk area nearest to the soil). All material coming from the same plant was included in a single paper bag properly labeled. The samples dried off in a. Heraeus Baureihe 6000 stove at temperature of 70°C during 72 hours. The dry material was weighed in an Mettler PE 360 analytic scale and the obtained data underwent a variance statistical analysis (ANOVA) and means comparison with the method of Tukey.
c) Total dry weight of the plant (includes weight of the frond and capsules). This parameter was determined adding the total weight of the frond and the total weight of the capsules.

The statistical analysis of the data obtained revealed that the CSb 5-4 and 5-18 transgenic lines that express the bacterial citrate synthase, accumulate more total dry weight of capsules than control plants (1522) in the treatment of 22 parts per million (ppm) of phosphorus (figure 4), The difference is significant with a 95% confidence interval, being the transgenic plants those accumulating higher biomass.

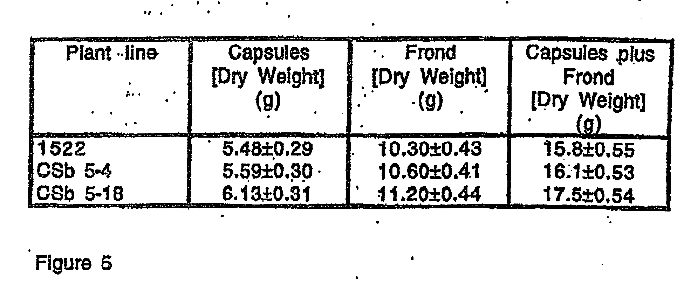

In the 44 ppm treatment, the transgenic line B5-18 accumulated more biomass than the transgenic CSb 5-4 and the control plant (1522). The difference in the dry weight of the capsules among the CSb 5-18 line and the control 1522 was statistically significant, with a 95% confidence interval (see figure 5).

The analysis of the total dry weight of the CSb transgenic plants and control plants, showed that in the treatments of 22 and 44 ppm there is significant difference among the plant lines evaluated with a 95% confidence interval. It was found that the CSb 5-18 transgenic line accumulates higher biomass in the plant than the CSb 5-4 transgenic line, and that both transgenic lines accumulated more plant biomass than the non-transformed control plant (see figure 4 and 5).

Analysis of the total dry weight of CSb transgenic and control plants, confirmed that the transgenic lines accumulate more biomass than the untransformed controls: 1) in the 22 ppm treatment, the two transgenic lines accumulated higher values of total biomass, these differences being statistically significant; 2) In the treatment of 44 ppm the CSb 5-18 transganic line accumulated significantly more biomass than the control plant. No significant differences were noted between the total dry weight of the CSb 5-4 line and the control in the 44 ppm treatment (see figures 4 and 5).

These results demonstrate that the transgenic lines expressing a cytoplasmic citrate synthase have an increased capacity of synthesis and exudation of organic acids and accumulate more biomass than their non transgenic counterparts when grown under conditions of low phosphate availability.

It is also important to point out that the transgenic line (CSb 5-18) that produces and exudes more citric acid, accumulated more biomass, thus demonstrating that the capacity of the plant to exude organic acids has a direct correlation with plant growth and biomass accumulation under conditions of low phosphate availability.

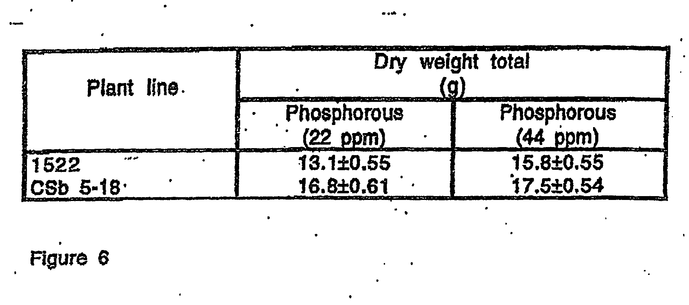

It is also important to point out that the biomass accumulated by the CSb 5-18 plants in the treatment supplemented with 22 ppm phosphorus, was the same or higher than the biomass accumulated by control plants grown at 44 ppm phosphorus (see figure 6). This demonstrates that the CSb 5-18 transgenic line is capable of accumulating as much or more biomass than an untransformed control plant grown in double the amount of fertilizer.

### 2. Tolerance of Tobacco plants containing increased synthesis, accumulation and citrate exudation levels at toxic levels of aluminum in pH acid.

Because the evidence of the role of citrate excretion in the tolerance to the aluminum is indirect, it was necessary to determine whether the CSb lines with high levels of citrate synthesis and excretion, were more tolerant than the control plants to phytotoxio aluminum concentrations. Since root growth inhbition is the first and most obvious effect of aluminum toxicity, we quantified the effect of increasing concentrations of aluminum on the root growth of CSb transgenic tobacco lines. To evaluate the effect of the aluminum on root growth, the vertical transfer technique was used (Taiz L. and Murphy A. (1995.). Plant Physiology. 108: 29-38.). This technique allowed us to determine the effect on root development of toxic compounds added to the nutrient solution from the moment of application.

In these experiments, 100 seeds of the T2 progeny of homozygote CSb and control lines were germinated on plates covered with filter paper and moistened by capilarity with Blaydes nutrient solution at pH 4.3. Seven days after germination, the plates containing the seedlings were. rotated 90 degrees and the nutrient solution replaced by nutrient solution containing different concentrations of aluminum at pH 4.3. After 7 days of growth in the presence of aluminum, the length of the root was quantified for each line. It was observed that the inhibition of root growth, at the different concentrations of aluminum tested, is significantly smaller for the CSb lines when compared to the control line. Statistical analysis of two independent experiments showed statistically significant differences between the, control and the CSb lines in all the evaluated concentrations (P 0.0001) with the exception of 50mM for which no significant difference was observed (see figure 5).

To evaluate whether citrate overproduction also had an effect on root development in seeds germinated directly in aluminum containing media (at pH 4.3), seeds of control and CSb lines were germinated in media containing aluminum at concentrations between 0.1 and 1 mM. It was observed that at concentrations over 300 mM, the control seeds germinated but did not develop roots. Closer inspection of control seedlings germinated at lower aluminum concentrations (50-75 mM), revealed that although root growth was only marginally inhibited, root hair development was severly impaired. When the CSb lines were germinated on aluminum containing media, it was noted that both root growth and root hair development was significantly less affected than in the controls subjected to the same treatment. It was also observed that the transgenic lines with higher citrate content were less susceptible to aluminum toxicity.

To corroborate that the aluminum-tolerance phenotype is due to the presence of the 35SCSb recombinant molecule in the genome of the transgenic tobacco lines, the, pattern of segregation of aluminum-tolerance was examined in the T1 progeny of the selected lines. It was observed that tolerant/susceptible phenotypes segregate with a 3:1 pattern in the lines containing single inserts, and that the tolerance of Aluminum co-segregates with the kanamycin resistance gene present in the T-DNA of the binary vector employed for the production of CSb lines. These observations confirm that the resistance phenotype is due to the presence of the 35S-CSb recombinant molecule in the genome of the transgenic tobacco analyzed.

### Example 3

General applicability of the technology

### 1. Application to different plant species

To demonstrate that the invention is applicable to other plant species different to tobacco, the T-DNA contained in the p35S-CSb recombinant molecule was introduced to the genome of Papaya plants by means of the particle bombardment transformation system (Cabrera-Ponce et. al. (1995), Plant Cell Reports 15: 1-7). Just as observed previously for tobacco, it was found that the Papaya lines presented 2 to 3 times higher levels of citrate Synthase activity than the controls transformed with the vector without the citrate synthase coding sequence. To test the aluminum-tolerance level of the CSb transgenic papaya lines, 20 root-less regenerated plants from each line were transferred to rooting media containing different aluminum concentrations. It was found that the development of the root was inhibited completely in the control plants exposed to concentrations of 50 mM aluminum or higher. under these conditions the control plants not only do not form roots, but were also unable to form new leaves or to expand the already existing ones. In contrast, it was found that the CSb papaya lines were able to form roots and grow normally in aluminum concentrations of up to 300 mM.

### Brief description of the drawings

Figure 1. Shows the steps followed to construct p35SCSb. The nucleotide and amino acid sequence at the translation start site of the original Pseudomonas aeruginosa citrate synthase gene (pPKB) and the resulting fusion derived from the cloning of the Bcl I-Bam HI fragment containing most of the bacterial citrate synthase coding sequence into the expression vector pB2, are shown.

Figure 2. Determination of citrate synthase activity . present in root extracts of CSb transgenic and control tobacco plants. It can be observed that the transgenic plants have an increased activity of citrate synthase with reference to the control.

Figure 3. Determination of the citrate levels present in root extracts of CSb transgenic and control plants. Also presented is the level of citrate exuded by CSb transgenic and the control tobacco plants. It can be noted that the transgenic plants have an increased capacity to accumulate and exude citric acid with respect to the control plants.

Figure 4. Determination of the biomass accumulated by transgenic tobacco plants expressing the Pseudomonas aeuroginosa citrate synthase coding sequence (CSb 5-4 and CSb 5-18) and control plants (1522) grown in presence of 22 parts per million of phosphorus. The accumulated biomass is presented as dry weight of capsules, frond and capsules plus frond. The results presented are the average and standard error of eight repetitions per treatment. The statistical analysis (ANOVA and Tukey tests), revealed a statistically significant difference between the biomass accumulated by the transgenic CSB lines and the controls, at the phosphorus concentrations tested.

Figure 5. Determination of the biomass accumulated by transgenic tobacco plants expressing the Pseudomonas aeuroginose citrate synthase coding sequence (CSb 5-4 and CSb 5-18) and control plants (1522) grown in presence of 44 parts per million of phosphorus. The accumulated biomass is presented as dry weight of capsules, frond and capsules plus frond. The results presented are the average and standard error of eight repetitions per treatment. The statistical analysis (ANOVA and Tukey test), revealed a significant increase in the yield of transgenic plants compared with the controls, at the phosphorus concentrations tested.

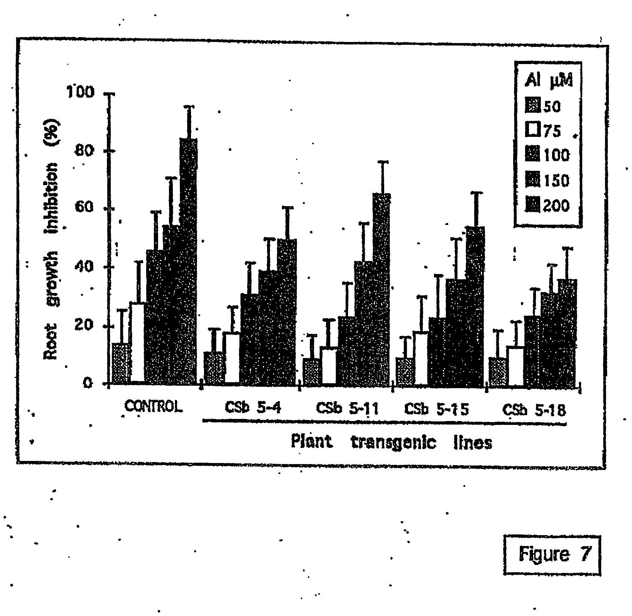

Figure 6. Comparison of the total biomass produced by the CSb 5-18 transgenic plants and the control plants, grown at 22 and 44 parts per million of phosphorus. Figure 7. Determination of the effect of increased concentrations of aluminum at pH 4.5 on the root growth of CSb and control plants. The effect is plotted as percentage of root growth inhibition in presence of aluminum with respect to the growth of the same plants in the absence of aluminum. It can be noted that the CSb transgenic plants roots grow better in the presence of toxic concentrations of aluminum.

## Claims

1. Method for obtaining transgenic plants having an increased capacity to synthesize, to accumulate and to exude organic acids, by integration into their genome of a recombinant heterologous DNA molecule encoding enzymes that synthesize organic acids, involving the following steps:
a) preparation of a recombinant heterologous DNA molecule encoding one or more genes for enzymes that synthesize organic acids, linked to a promoter sequence functional in plants, and to a transcrition termination/polyadenylation sequence functional in plants;
b) the transformation of plant cells with the recombinant DNA molecule, and
c) the regeneration of transgenic plants starting from transformed cells, or of seeds from plants obtained from these transformed cells, for one or several generations, wherein the genetic information of these transformed cells, includes the recombinant DNA molecule coding for enzymes that synthesize organic acids.

2. The method according to claim 1, in which the .recombinant DNA molecule comprises one or more microbial genes coding for enzymes that synthesize organic acids.

3. The method according to claim 1, wherein the recombinant DNA molecule comprises a gene of plant origin coding for an enzyme that synthesizes organic acids.

4. The method according to claim 1, wherein the recombinant DMA molecule comprises a gene of animal origin coding for an enzyme that synthesizes organic acids.

5. The method according to claim 2, wherein the recombinant DNA molecule comprises one or more bacterial genes that code for an enzyme that synthesizes organic acids.

6. The method according to claim 1, wherein the recombinant molecule comprises a gene that codes for the enzyme Citrate Synthase.

7. The method according to claim 1, wherein the recombinant molecule comprises a gene that codes for the enzyme Malate dehydrogenase.

8. The method according to claim 1, wherein the enzyme that synthesizes organic acids is located in the cytoplasm.

9. The method according to claim 1, wherein the enzyme that synthesizes organic acids is located in chloroplasts.

10. The method according to claim 1, wherein the enzyme that synthesizes organic acids is located in the mitochondria.

11. The method according to claim 5, wherein the recombinant molecule comprises a gene of Pseudomonas aeruginosa that codes for Citrate Synthase.

12. The method according to claims 1 - 10, wherein the transcription termination sequence is the transcription termination sequence of the Nopaline Synthetase gene.

13. The method according to claims 1 - 10, wherein the promoter is a constitutive promoter.

14. The method according to claims 1 - 10, wherein the promoter is a root-specific promoter.

15. The method according to claims 1 - 10, wherein the promoter is a promoter inducible by stress caused by low Phosphate availability.

16. The method according to claims 1 - 10, wherein the promoter is a promoter inducible by stress caused,by low. Iron availability.

17. The method according to claims 1, - 10, wherein the promoter is the 35S promoter of the cauliflower mosaic virus.

18. The method according to claims-9 and 10, wherein the recombinant molecule comprises a signal peptide sequence to direct a heterologous enzyme that synthesizes organic-acids to the chloroplast or the mitochondria of the transgenic cells.

19. A recombinant heterologous DNA molecule comprising one or more genes that code for enzymes that synthesize organic acids, functionally linked to a promoter sequence functional in plants, and to a transcription termination/polyadenylation sequence functional in plants.

20. The recombinant DNA molecule according to claim, 19, wherein the coding sequence for the enzyme that synthesizes organic acids, is from one or more microbial genes.

21. The recombinant DNA molecule according to claim 19, wherein the coding sequence for the enzyme that synthesizes organic acids is from a gene of plant origin.

22. The recombinant DNA molecule according to claim 19; wherein the coding sequence for the enzyme that synthesizes organic acids is from a gene of animal origin.

23. The recombinant DNA molecule according to claim 19, wherein the coding sequence for the enzyme that synthesizes organic acids, is from one or more bacterial genes.

24. The recombinant DNA molecule according to claim 19, wherein the gene that codes for the enzyme that synthesizes organic acids' is a gene that codes for the enzyme citrate synthase.

25. The recombinant DNA molecule according to claim 19, wherein the gene that .codes for the enzyme that synthesizes organic acids is a gene of Pseudomonas aeruginosa that codes . for the enzyme citrate synthase.

26. The recombinant DNA molecule according to claim 19, wherein the gene that codes for the enzyme that synthesizes organic acids is' a gene that codes for the enzyme Malate deshydrogenase.

27. The recombinant DNA molecule according to claim 19, wherein the gene that codes for the enzyme that synthesizes organic acids is an enzyme that is located in the cytoplasm.

28. The recombinant DNA molecule according to claim 19, wherein the gene that codes for the enzyme that synthesizes organic acids is an enzyme that is located in the chloroplast.

29. The recombinant DNA molecule according to claim 19, wherein the promoter is a constitutive promoter.

30. The recombinant DNA molecule according to claim 19, wherein the promoter is a root-specific promoter.

31. The recombinant DNA molecule according to claim 19, wherein the promoter is a promoter whose expression is inducible by stress caused by, low phosphate availability.

32. The recombinant DNA molecule according to claim 19, wherein the promoter is a promoter whose expression, is inducible by stress caused by low iron availability.

33. The recombinant DNA molecule according to claim 19, wherein the promoter is the 35S promoter of the cauliflower mosaic virus.

34. The recombinant DNA molecule according to claim 19 comprising a sequence that includes a transit peptide sequence for chloroplast or mitochondrial protein targeting in plants.

35. The recombinant DNA molecule according to claim 19, comprising a transcription termination/polyadenylation sequence that is the transcription termination/polyadenylation sequence of the Nopaline Synthetase gene.

36. The recombinant DNA molecule according to claim 19, as defined in figure 1.

37. The vector comprising the recombinant DNA molecule according to claim 19.

38. Transgenic plants with increased capacity to synthesize, to accumulate and to exude organic acids by integration into their genome of a recombinant heterologous DNA molecule as defined in any of claims 19 to 36.

39. Transgenic plants according to claim 38, tolerant to toxic concentrations of Aluminum.

40. Transgenic plants according to claim 38, having increased capacity to solubilize or accumulate phosphate.

41. Transgenic plants according to claim 38, having increased capacity to solubilize or accumulate iron.

42. Transgenic plants according to claim 38, requiring less fertilizer for their growth.

43. Transgenic plants according to claim 38, that develop better or have higher productivity in acid soils.

44. The transgenic plants according to claim 38, wherein the plant is a monocotyledonous plant.

45. Transgenic plants according to claim 38, wherein the plant is a dicotyledonous plant.

46. Transgenic plants according to claim 44, wherein the plant belongs to anyone of the families: Poaceae or Lileaceae.

47. Transgenic plants according to claim 45, wherein the plant belongs to anyone of the families: Leguminoseae, Solanaceae, Caricaceae or Cucurbitaceae.

48. Transgenic plants according to claim 44, wherein the plant belongs to any of the species: Triticum spp, Oryza sativa, Zea mays, Sorghum bicolor, Avena sativa or Saccharum officcianarum.

49. Transgenic plants according to claim 45, wherein the plant belongs to any of the species: Solanum tuberosum, Lycopersicum esculentum or Glycine max.

50. Transgenic plants according to claim 45, wherein the plant is of the Nicotiana genus.

51. Transgenic plants according to claim 50, wherein the plant is of the Nicotiana tabacum species.

52. Transgenic plants according to claim 45, wherein the plant is of the Carica genus.

53. Transgenic plants according to claim 52, wherein the plant is of the Carica papaya species.

54. Use of the transginic plants according to claim 26 in acid soils.

55. Use of the transgenic plants according to claim 26 in soils containing-phosphates in forms not available for the plant nutrition.

56. Use of the transgenic plants according to claim 26 for practice or cultivation systems that use less fertilizer.

57. The transgenic seeds or any vegetative reproductive structure attainable from a transgenic plant as defined in the claim 26.

58. A transformed cell or protoplast transformed with the recombinant DNA molecule as defined in any of claims 19 to 36.
